# EUROPÄISCHE PATENTSCHRIFT

(11) **EP 1 701 661 B1**
(45) Veröffentlichungstag und Bekanntmachung des Hinweises auf die Patenterteilung: **28.09.2011**
(21) Anmeldenummer: 05707544.2
(22) Anmeldetag: 21.02.2005
(51) Int. Cl.: A61B 17/32, A61B 17/28

(54) **MEDIZINISCHES SCHNEID- UND/ODER HALTEINSTRUMENT**
MEDICAL CUTTING AND/OR HOLDING INSTRUMENT
INSTRUMENT MEDICAL DE DECOUPAGE ET/OU DE MAINTIEN

(30) Priorität: 25.02.2004 DE 102004009200
(43) Veröffentlichungstag der Anmeldung: 20.09.2006
(73) Patentinhaber: Karl Storz GmbH & Co. KG, 78532 Tuttlingen (DE)
(72) Erfinder: STORZ, Martin, 78532 Tuttlingen (DE)
(74) Vertreter: Hofmeister, Frank
(86) Internationale Anmeldenummer: PCT/EP2005/001763
(87) Internationale Veröffentlichungsnummer: WO 2005/079679

(56) Entgegenhaltungen:
- EP-A- 1 365 691
- EP-A- 1 365 691
- EP-A1- 0 541 930
- DE-A1- 10 156 917
- DE-A1- 19 910 457
- DE-C- 356 185
- DE-C- 356 185
- DE-U1- 9 421 125
- DE-U1- 9 421 125
- DE-U1- 20 311 293
- DE-U1- 20 311 293
- US-A- 1 754 806
- US-A- 1 754 806
- US-A- 3 404 677
- US-A- 3 404 677
- US-A- 5 507 772
- US-A- 5 766 177
- US-A- 5 766 177
- US-A- 5 851 214
- US-A- 5 851 214
- US-A1- 2002 151 931
- US-A1- 2002 151 931

## Beschreibung

Die Erfindung betrifft ein medizinisches Schneid- und/oder Halteinstrument mit einem Schaft, einer am proximalen Ende des Schaftes angeordneten, aus zwei Griffteilen bestehenden Handhabe sowie einer in einer Führungsnut des Schaftes gelagerten Zug-/Schubstange zum Betätigen eines am distalen Ende des Schaftes angeordneten, aus zwei Maulteilen bestehenden Werkzeugs, wobei die Zug-/Schubstange zum Öffnen und Schließen des Werkzeugs distalseitig mit mindestens einem verschwenkbaren Maulteil des Werkzeugs und proximalseitig mit einem verschwenkbaren Griffteil der Handhabe koppelbar ist und wobei die Zug-/Schubstange und das verschwenkbare Griffteil über einen Kopplungsmechanismus lösbar miteinander verbindbar sind.

Medizinische Schneid- und/oder Halteinstrumente werden sowohl in der offenen als auch in der endoskopischen Chirurgie vielfältig eingesetzt. Aufgrund der verwendungsbedingten sehr schlanken Bauweise endoskopischer Instrumente, die in der Regel durch hohle Trokarhülsen in das Operationsgebiet eingeführt werden, haben sich in der endoskopischen Chirurgie sogenannte Rohrschaftinstrumente für die Ausgestaltung von Schneid- und/oder Halteinstrumenten etabliert und bewährt. Diese Rohrschaftinstrumente zeichnen sich dadurch aus, dass der Instrumentenschaft als hohles Rohr ausgebildet ist und die zur Betätigung des distalseitigen Maulteils vorgesehene Zug-/Schubstange verschiebbar im Inneren des hohlen Schaftrohres gelagert ist.

Da insbesondere mit Blut in Kontakt kommende medizinische Instrumente, die nicht als Einweg-Instrumente ausgelegt sind, einer gründlichen Reinigung und Sterilisation bedürfen, müssen diese Rohrschaftinstrumente zerlegbar ausgebildet sein, um insbesondere eine zuverlässige Reinigung des hohlen Schaftrohres zu gewährleisten. Diese konstruktiven Maßnahmen, die das Zerlegen des medizinischen Instruments in wenigstens drei Teile, nämlich das hohle Schaftrohr, die Zug-/Schubstange sowie die Handhabe, ermöglichen, verteuern die Herstellung derartiger medizinischer Instrumente und erschweren deren Montage und Demontage. Aus der DE 94 21 125 U1 ist ein gattungsgemäßes medizinisches Schneid- und/oder Halteinstrument bekannt, bei dem die Zug-/Schubstange in einer offenen Führungsnut des Schaftes gelagert ist und die Zug-/Schubstange zu Reinigungszwecken aus dieser Führungsnut des Schaftes herausklappbar ist. Diese Ausgestaltung des medizinischen Instruments erleichtert die Reinigung sowie die Montage und Demontage des Instruments deutlich. Darüber hinaus ist diese konstruktive Ausgestaltung deutlich einfacher und somit auch kostengünstiger zu verwirklichten als dies bei den bekannten Rohrschaftinstrumenten möglich ist.

Nachteilig bei dieser bekannten Konstruktion ist jedoch, dass zumindest die Kopplung der Zug-/Schubstange mit dem verschwenkbaren Maulteil des Werkzeugs eine vertikale Verlagerung der Zug-/Schubstange quer zur Bewegungsrichtung der Zug-/Schubstange bewirkt, wenn die Zug-/Schubstange das Maulteil zwischen der geschlossenen und der geöffneten Stellung verschwenkt. Aufgrund dieser Vertikalverlagerung, bei der die Zug-/Schubstange aus der Führungsnut des Schaftes heraustritt, ist ein derartiges medizinisches Instrument nicht für den Einsatz in der endoskopischen Chirurgie geeignet, da dies zu einem Verklemmen innerhalb der Trokarhülse führen kann.

US-A-5 851 214 offenbart ein Instrument gemäß dem Oberbegriff des Anspruchs 1.

Davon ausgehend liegt der Erfindung die **Aufgabe** zugrunde, ein medizinisches Schneid- und/oder Halteinstrument der eingangs genannten Art so auszugestalten, dass dieses auch für endoskopische Zwecke verwendbar ist.

Die **Lösung** dieser Aufgabenstellung ist erfindungsgemäß dadurch gekennzeichnet, dass die Zug-/Schubstange sowohl proximalseitig als auch distalseitig mit Spiel in einer Richtung im wesentlichen quer zur Bewegungsrichtung der Zug-/Schubstange mit den verschwenkbaren Bauteilen gekoppelt ist, wobei der kopplungsmechanismus zum lösbaren Verbinden der Zug/Schubstange mit dem verschwenkbaren Griffteil als in Langlöchern geführte Schraubverbindung ausgebildet ist.

Aufgrund der erfindungsgemäßen beidseitig spielbehafteten Kopplung der Zug-/Schubstange ist es möglich, dass das Verschwenken des Maulteils über eine Betätigung des verschwenkbaren Griffteils der Handhabe ausschließlich eine Axialverschiebung der Zug-/Schubstange innerhalb der Führungsnut des Schaftes bewirkt. Das in einer Richtung im wesentlichen quer zur Bewegungsrichtung der Zug-/Schubstange vorgesehene Kopplungsspiel ermöglicht es den jeweils auf einer Bogenbahn verlaufenden Kopplungspunkten der angeschlossenen Bauteile eben diesen Bogenbahnen zu folgen, jedoch ohne dabei eine Vertikalverlagerung der Zug-/Schubstange zu bewirken. Ein solchermaßen ausgestaltetes medizinisches Schneid- und/oder Halteinstrument lässt sich auch problemlos in eine enge Trokarhülse einsetzen und ist somit für den Einsatz in der endoskopischen Chirurgie geeignet.

Gemäß einer praktischen Ausführungsform der Erfindung wird vorgeschlagen, dass die Lagerstelle an jeweils einem der miteinander zu koppelnden Bauteile zur Ausbildung des beidseitigen Kopplungsspiels als Langloch ausgebildet ist.

Um die Kopplung der Zug-/Schubstange mit dem verschwenkbaren Griffteil zu vereinfachen, wird mit der Erfindung weiterhin vorgeschlagen, dass das distalseitige Langloch als offene Rastnut ausgebildet ist, die ein einfaches Einhängen und wieder Aushängen der Zug-/Schubstange aus der Kopplung mit dem Griffteil ermöglicht.

Gemäß der Erfindung ist der Kopplungsmechanismus zum lösbaren Verbinden der Zug-/Schubstange mit dem verschwenkbaren Griffteil als Schraubverbindung ausgebildet. Die Ausbildung einer Schraubverbindung stellt eine besonders einfach herzustellende und handzuhabende Art der Kopplung zwischen zwei Bauteilen dar.

Zur Ausbildung der Schraubverbindung ist das stangenseitige Ende des verschwenkbaren Griffteils zur Aufnahme der Zug-/Schubstange vorzugsweise gabelförmig ausgebildet und ist in der Zug-/Schubstange oder im stangenseitigen Ende des verschwenkbaren Griffteils eine Gewindebuchse zur Aufnahme eines Gewindestifts und im jeweils anderen Bauteil das Langloch zur führenden Aufnahme des Gewindestifts ausgebildet.

Gemäß einer zweiten nicht erfindungsgemäßen Ausführungsform zur Ausbildung des Kopplungsmechanismus zum lösbaren Verbinden der Zug-/Schubstange mit dem verschwenkbaren Griffteil wird vorgeschlagen, dass der Kopplungsmechanismus zum lösbaren Verbinden der Zug-/Schubstange mit dem verschwenkbaren Griffteil als Zapfen-Schlitz-Steuerung ausgebildet ist, die einen an der Zug-/Schubstange oder am stangenseitigen Ende des verschwenkbaren Griffteils angeordneten Kopplungsstift und eine im jeweils anderen Bauteil ausgebildete offene Rastnut zur führenden Aufnahme des Kopplungsstifts umfasst. Durch die Ausbildung des distalseitigen Langlochs als offene Rastnut lässt sich die Zug-/Schubstange zu Reinigungszwecken einfach und schnell aus der Führungsnut des Schaftes herausklappen.

Um die Zug-/Schubstange in der mit dem verschwenkbaren Griffteil gekoppelten Arbeitsstellung zu sichern und ein versehentliches Lösen der Kopplung zu vermeiden, wird vorgeschlagen, dass Zug-/Schubstange in der mit dem verschwenkbaren Griffteil gekoppelten Arbeitsstellung über ein Rastelement fixierbar ist, wobei das Rastelement vorzugsweise zusätzlich über ein Federelement in Richtung der verrastenden Stellung vorgespannt ist.

Zusätzlich zu der Verwendung bei medizinischen Schneid- und/oder Halteinstrumenten mit einem verschwenkbaren Griffteil der Handhabe werden auch gattungsgemäße medizinische Schneid- und/oder Halteinstrumente mit einem axial in Schaftrichtung verschiebbaren Griffteil vorgeschlagen. Um auch bei einem mit einem axial verschiebbaren Griffteil versehenen medizinischen Instrument dessen Verwendung für die endoskopische Chirurgie zu ermöglichen, wird vorgeschlagen, dass die Zug-/Schubstange distalseitig mit Spiel in einer Richtung im wesentlichen quer zur Bewegungsrichtung der Zug-/Schubstange mit dem verschwenkbaren Maulteil gekoppelt ist.

Bei dieser Ausgestaltungsform ist es ausreichend nur die distalseitige Kopplung mit Spielfreiheit auszustatten, da das Griffteil ausschließlich axial verschiebbar am Instrumentenschaft gelagert ist.

Weiterhin wird gemäß einer praktischen Ausführungsform vorgeschlagen, dass bei dieser Ausgestaltungsform der Kopplungsmechanismus zum lösbaren Verbinden der Zug-/Schubstange mit dem verschiebbaren Griffteil als im wesentlichen quer zur Bewegungsrichtung der Zug-/Schubstange ausgerichteter Mitnahmestift ausgebildet ist, der einerseits an der Zug-/Schubstange gelagert ist und sich andererseits am verschiebbaren Griffteil abstützt.

Gemäß einer bevorzugten Ausführungsform ist im Schaft ein Langloch zur führenden Aufnahme des Mitnahmestifts ausgebildet und der Mitnahmestift vorzugsweise als mit einer Riegelplatte versehener drehbarer Stift ausgebildet.

Schließlich wird vorgeschlagen, dass das verschiebbare Griffteil über ein an dem Mitnahmestift angreifendes Federelement in die das Werkzeug öffnende Stellung vorgespannt ist, um das Öffnen des Werkzeugs entgegen der durch das Zusammendrücken der Griffteile bewirkte Schließen des Werkzeugs zu erleichtern.

Weitere Merkmale und Vorteile der Erfindung ergeben sich anhand der zugehörigen Zeichnung, in der vier Ausführungsbeispiele eines erfindungsgemäßen medizinischen Schneid- und/oder Halteinstruments nur beispielhaft schematisch dargestellt sind. In der Zeichnung zeigt:
- Fig. 1: eine teilweise geschnittene Seitenansicht einer ersten Ausführungsform eines erfindungsgemäßen medizinischen Schneid- und/oder Halteinstruments;
- Fig. 2a: eine Seitenansicht einer zweiten Ausführungsform eines erfindungsgemäßen medizinischen Schneid- und/oder Halteinstruments;
- Fig. 2b: eine vergrößerte und teilweise geschnittene Detailansicht des medizinischen Instruments gemäß Fig. 2a;
- Fig. 3: einen ausschnittweisen Schnitt entlang der Linie III-III gemäß Fig. 2b;
- Fig. 4a: eine geschnittene Seitenansicht einer dritten Ausführungsform eines erfindungsgemäßen medizinischen Schneid- und/oder Halteinstruments;
- Fig. 4b: eine vergrößerte Detailansicht des Details IVb gemäß Fig. 4a und
- Fig. 5: eine geschnittene Seitenansicht einer vierten Ausführungsform eines nicht erfindungsgemäßen medizinischen Schneid- und/oder Halteinstruments.

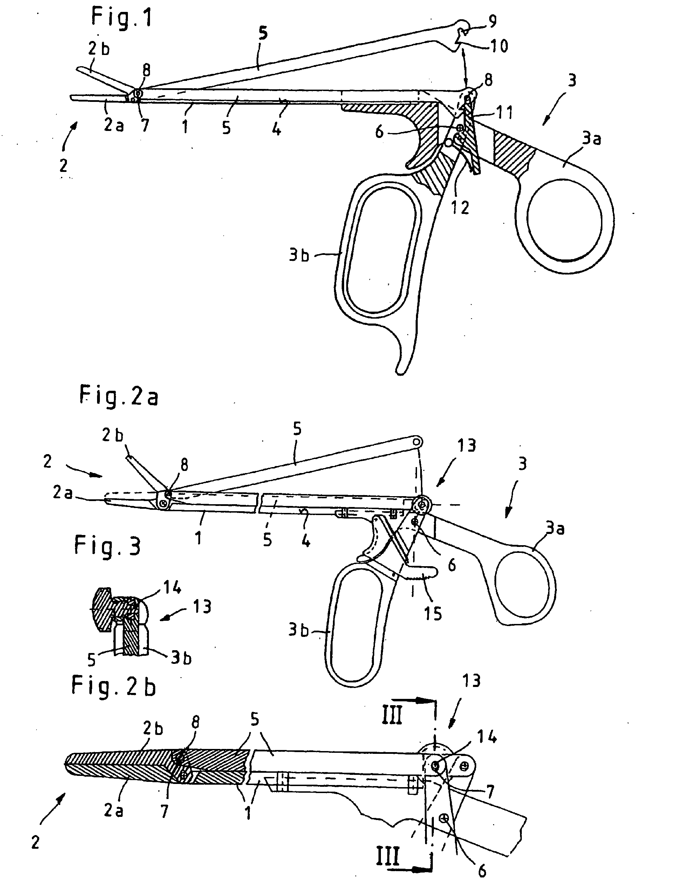

Die in den Abbildungen Fig. 1, 2a, 4a und 5 dargestellten medizinischen Schneid- und/oder Halteinstrumente bestehen im Wesentlichen aus einem stangenförmigen Schaft 1, einem am distalen Ende des Schaftes 1 angeordneten, aus zwei Maulteilen 2a und 2b bestehenden Werkzeug 2, einer am proximalen Ende des Schaftes 1 angeordneten, mit zwei Griffteilen 3a und 3b versehenen Handhabe 3 sowie einer formbündig in einer Führungsnut 4 des Schaftes 1 axial verschiebbar gelagerten Zug-/Schubstange 5 zur Betätigung des Werkzeugs 2 mittels der Handhabe 3.

Wie weiterhin aus den Abbildungen ersichtlich, besteht das Werkzeug 2 aus einem starren, fest mit dem Schaft 1 verbundenen Maulteil 2a sowie einem über die Zug-/Schubstange 5 verschwenkbaren Maulteil 2b. Ebenso weist die Handhabe 3 ein starres, fest mit dem Schaft 1 verbundenes Griffteil 3a sowie ein gegenüber dem starrem Griffteil 3b verstellbares Griffteil 3b auf. Das verschwenkbare Maulteil 2b und das verstellbare Griffteil 3b sind über die Zug-/Schubstange 5 miteinander gekoppelt, wobei die Zug-/Schubstange 5 und das verschwenkbare Griffteil 3b über einen Kopplungsmechanismus lösbar so miteinander verbindbar sind, dass die Zug-/Schubstange 5, beispielsweise zu Reinigungszwecken, nach oben aus der Führungsnut 4 des Schaftes 1 herausgeschwenkt werden kann.

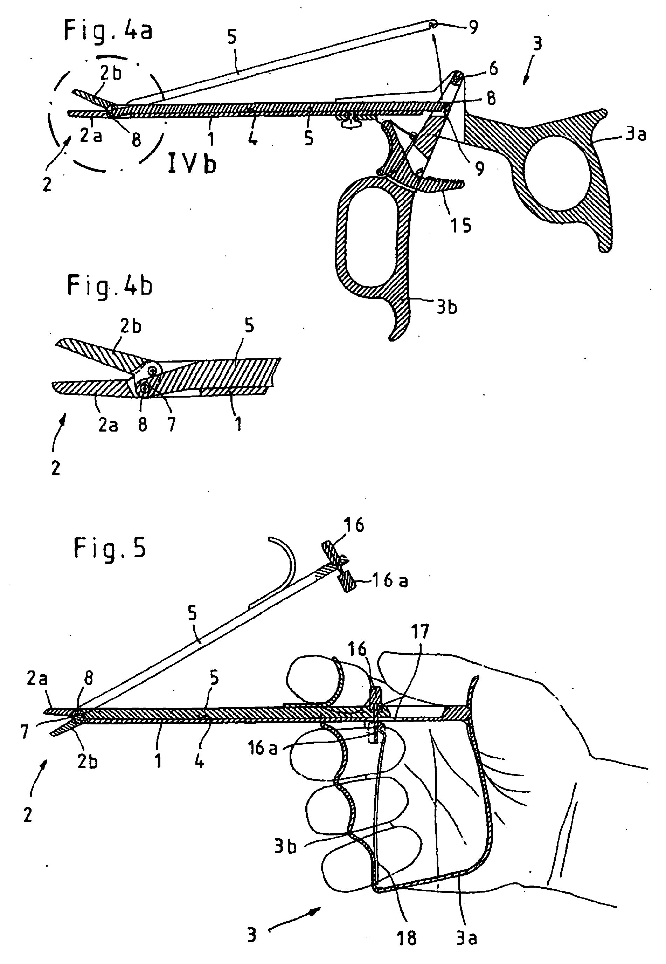

Während bei den in den Abbildungen Fig. 1 bis 4b dargestellten medizinischen Instrumenten das verstellbare Maulteil 3b um eine Schwenkachse 6 gegenüber dem starren Griffteil 3a verschwenkbar ist, ist in Fig. 5 eine Ausführungsform dargestellt, bei der das verstellbare Griffteil 3b in Schaftrichtung axial verschiebbar ausgebildet ist.

Um zu verhindern, dass die Zug-/Schubstange 5 beim Betätigen des Werkzeugs 2 über das verschwenkbare Griffteil 3b der Handhabe 3 nach oben aus der Führungsnut 4 des Schaftes 1 herausgehoben wird, da die Lagerstellen sowohl des verschwenkbaren Maulteils 2b als auch des verschwenkbaren Griffteils 3b bei ihrer Verlagerung zwischen den beiden Endstellungen eine Bogenbahn beschreiben, sind bei den in den Abbildungen Fig. 1 bis 4b dargestellten Ausführungsformen die beidseitigen Lagerstellen der Zug-/Schubstange 5 mit Kopplungsspiel ausgebildet.

Dieses Kopplungsspiel wird bei diesen Ausführungsformen dadurch erzielt, dass die beidseitigen Lagerstellen der Zug-/Schubstange 5 als Langlöcher 7 ausgebildet sind, in die entsprechende Kopplungsstifte 8 des verschwenkbaren Maulteils 2b sowie des verschwenkbaren Griffteils 3b eingreifen. Wie weiterhin aus den Abbildungen Fig. 1 und 4a ersichtlich, ist es auch möglich, das proximalseitige Langloch 7 als offene Rastnut 9 auszubilden, die ein besonders einfaches und schnelles Koppeln von Zug-/Schubstange 5 und verschwenkbarem Griffteil 3b ermöglicht.

Die Spielfreiheit, die die Kopplungsstifte 8 in den Langlöchern 7 haben ermöglicht es, dass das Betätigen des verschwenkbaren Griffteils 3b eine ausschließliche Axialverschiebung der Zug-/Schubstange 5 bewirkt, weshalb ein solchermaßen ausgebildetes medizinisches Schneid- und/oder Halteinstrument auch verklemmungsfrei in einer Trokarhülse betätigbar ist, wodurch dieses Instrument auch für die endoskopische Chirurgie verwendbar ist.

Alternativ zu der dargestellten Anordnung der Langlöcher 7 an der Zug-/Schubstange 5 und der Kopplungsstifte 8 an den verschwenkbaren Bauteilen 2b und 3b ist es selbstverständlich auch möglich, die Langlöcher 7 an den verschwenkbaren Bauteilen 2b und 3b und die Kopplungsstifte 8 an der Zug-/Schubstange 5 auszubilden.

Gerade bei der Verwendung dieser medizinischen Schneid- und/oder Halteinstrumente für die endoskopische Chirurgie ist es vorteilhaft, wenn der Schaft 1 mit der formbündig in die Führungsnut 4 eingesetzten Zug-/Schubstange 5 einen runden Querschnitt aufweist, da diese Querschnittsform das Abdichten des Schaftes 1 in einer Trokarhülse, beispielsweise durch eine den Schaft 1 umschließende Ringdichtung, zur Aufrechterhaltung des Insufflationsdrucks erleichtert. Selbstverständlich sind aber auch alle anderen Querschnittsformen möglich, bei denen die in die Führungsnut 4 eingesetzte Zug-/Schubstange 5 die Außenkontur des Schaftes 1 formbündig ergänzt.

Neben der unterschiedlichen Art zur Verlagerung des verstellbaren Griffteils 3b der Handhabe 3, nämlich als verschwenkbares Griffteil 3b gemäß Fig. 1 bis 4b oder als axial verschiebbares Griffteil 3b gemäß Fig. 5, unterscheiden sich die in den Abbildungen Fig. 1 bis 5 dargestellten Ausführungsformen medizinischer Schneid- und/oder Halteinstrumente durch die Art der Ausbildung des Kopplungsmechanismus zum lösbaren Verbinden der Zug-/Schubstange 5 mit dem verstellbaren Griffteil 3b.

Bei der in Fig. 1 dargestellten ersten Ausführungsform ist der Kopplungsmechanismus zum lösbaren Verbinden der Zug-/Schubstange 5 mit dem verschwenkbaren Griffteil 3b als Zapfen-Schlitz-Steuerung ausgebildet ist, die einen am stangenseitigen Ende des verschwenkbaren Griffteils 3b angeordneten Kopplungsstift 8 und eine am proximalen Ende der Zug-/Schubstange 5 ausgebildete offene Rastnut 9 zur führenden Aufnahme des Kopplungsstifts 8 umfasst.

Die Abbildung zeigt die Zug-/Schubstange 5 einmal in der nach oben aus der Führungsnut 4 des Schaftes 1 herausgeschwenkten Montage- und Reinigungsstellung und einmal in der mit dem verschwenkbaren Griffteil 3b gekoppelten Arbeitsstellung.

Um die Zug-/Schubstange 5 in der gekoppelten Arbeitsstellung zu sichern und ein versehentliches entkoppeln zu verhindern, ist die Zug-/Schubstange 5 bei der dargestellten Ausführungsform über ein in eine Rastkerbe 10 eingreifendes Rastelement 11 in der gekoppelten Stellung fixierbar, wobei das Rastelement 11 über ein Federelement 12 in Richtung der verrastenden Stellung vorgespannt ist.

Zum Lösen der Kopplung von Zug-/Schubstange 5 und Griffteil 3b ist es zunächst erforderlich, das Rastelement 11 gegen die Kraft des Federelements 12 außer Eingriff mit der Rastkerbe 10 zu verschwenken. Anschließend kann die Zug-/Schubstange 5 nach oben aus der Führungsnut 4 des Schaftes 1 herausgeklappt werden.

Bei der in den Abbildungen Fig. 2a bis 3 dargestellten zweiten Ausführungsform ist der Kopplungsmechanismus zum lösbaren Verbinden der Zug-/Schubstange 5 mit dem verschwenkbaren Griffteil 3b als Schraubverbindung 13 ausgebildet. Zur Ausbildung der Schraubverbindung 13 ist das stangenseitige Ende des verschwenkbaren Griffteils 3b zur Aufnahme der Zug-/Schubstange 5 gabelförmig ausgebildet und in der Zug-/Schubstange 5 eine Gewindebuchse zur Aufnahme eines Gewindestifts 14 der Schraubverbindung 13 ausgebildet. In den beiden gabelförmigen Enden des verschwenkbaren Griffteils 3b sind entsprechende Langlöcher 7 zur führenden Aufnahme des Gewindestifts 14 ausgebildet, wie dies insbesondere den Abbildungen Fig. 2b und 3 zu entnehmen ist.

Wie weiterhin aus Fig. 2a ersichtlich, ist das verschwenkbare Griffteil 3b der Handhabe 3 über eine vorzugsweise federbelastete Arretiervorrichtung 15 in der gegenüber dem starren Griffteil 3a verschwenkten Stellung fixierbar.

Bei der in Fig. 4a und 4b dargestellten dritten Ausführungsform ist der Kopplungsmechanismus zum lösbaren Verbinden der Zug-/Schubstange 5 mit dem verschwenkbaren Griffteil 3b wiederum als Zapfen-Schlitz-Steuerung ausgebildet. Im Gegensatz zu der in Fig. 1 dargestellten ersten Ausführungsform wird die Zug-/Schubstange 5 bei dieser Ausgestaltungsform nicht über ein Rastelement 11 in der gekoppelten Stellung fixiert. Darüber hinaus unterscheiden sich die beiden Ausführungsformen durch die Art der Lagerung der beiden Griffteile 3a und 3b aneinander sowie durch die Lage der Lagerstelle der Zug-/Schubstange 5 am verschwenkbaren Maulteil 2b.

Die in Fig. 5 dargestellte vierte Ausführungsform zeigt schließlich ein medizinisches Schneid- und/oder Halteinstrument mit einem axial verschiebbaren Griffteil 3b. Da bei dieser Ausgestaltungsform die proximalseitige der Lagerstelle der Zug-/Schubstange 5 aufgrund der Axialverschiebbarkeit des Griffteils 3b unproblematisch ist, ist es ausreichend, dass die Zug-/Schubstange 5 distalseitig mit Spiel in einer Richtung im wesentlichen quer zur Bewegungsrichtung der Zug-/Schubstange 5 mit dem verschwenkbaren Maulteil 2b gekoppelt ist, um ein Herausheben der Zug-/Schubstange 5 aus der Führungsnut 4 des Schaftes 1 zu verhindern.

Bei dieser Ausgestaltungsform ist der Kopplungsmechanismus zum lösbaren Verbinden der Zug-/Schubstange 5 mit dem verschiebbaren Griffteil 3b als im wesentlichen quer zur Bewegungsrichtung der Zug-/Schubstange 5 ausgerichteter Mitnahmestift 16 ausgebildet ist, der einerseits an der Zug-/Schubstange 5 gelagert ist und sich andererseits am verschiebbaren Griffteil 3b abstützt.

Wie aus der Abbildung ersichtlich, ist im Schaft 1 ein Langloch 17 zur führenden Lagerung des Mitnahmestifts 16 ausgebildet. Zum Festlegen der Zug-/Schubstange 5 in der gekoppelten Stellung weist der Mitnahmestift 16 eine Riegelplatte 16a auf, die nach dem Einsetzen in das Langloch 17 des Schaftes 1 um 90° verdrehbar ist und so ein Anheben der Zug-/Schubstange 5 blockiert. Die Riegelplatte 16a dient bei der dargestellten Ausführungsform weiterhin als Anschlagfläche für ein Federelement 18, über das das verschiebbare Griffteil 3b in die das Werkzeug 2 öffnende Stellung vorgespannt ist.

Die wie dargestellt ausgebildeten medizinischen Schneid- und/oder Halteinstrumente zeichnen sich dadurch aus, dass die formbündig in der Führungsnut 4 des Schaftes 1 gelagerte Zug-/Schubstange 5 einerseits einfach und schnell zu Montage- und Reinigungszwecken aus der Führungsnut 4 herausklappbar ist und andererseits das Verlagern der Zug-/Schubstange 5 über die verstellbaren Griffteile 3b der Handhabe 3 aufgrund der spielbehafteten Lagerung der Zug-/Schubstange 5 ein ausschließlich in Axialrichtung des Schaftes 1 verlaufende Verlagerung der Zug-/Schubstange 5 bewirkt. Aus diesem Grund sind solchermaßen ausgebildete medizinische Instrumente auch klemmfrei in engen Trokarhülsen betätigbar, wie dies in der endoskopischen Chirurgie verwendet werden.

### Bezugszeichenliste

- 1: Schaft
- 2: Werkzeug
- 2a: starres Maulteil
- 2b: verschwenkbares Maulteil
- 3: Handhabe
- 3a: starres Griffteil
- 3b: verstellbares Griffteil
- 4: Führungsnut
- 5: Zug-/Schubstange
- 6: Schwenkachse
- 7: Langloch
- 8: Kopplungsstift
- 9: Rastnut
- 10: Rastkerbe
- 11: Rastelement
- 12: Federelement
- 13: Schraubverbindung
- 14: Gewindestift
- 15: Arretiervorrichtung
- 16: Mitnahmestift
- 16a: Riegelplatte
- 17: Langloch
- 18: Federelement

## Patentansprüche

1. Medizinisches Schneid- und/oder Halteinstrument mit einem Schaft (1), einer am proximalen Ende des Schaftes (1) angeordneten, aus zwei Griffteilen (3a, 3b) bestehenden Handhabe (3) sowie einer in einer Führungsnut (4) des Schaftes (1) gelagerten Zug-/Schubstange (5) zum Betätigen eines am distalen Ende des Schaftes (1) angeordneten, aus zwei Maulteilen (2a, 2b) bestehenden Werkzeugs (2), wobei die Zug-/Schubstange (5) zum Öffnen und Schließen des Werkzeugs (2) distalseitig mit mindestens einem verschwenkbaren Maulteil (2b) des Werkzeugs (2) und proximalseitig mit einem verschwenkbaren Griffteil (3b) der Handhabe (3) koppelbar ist und wobei die Zug-/Schubstange (5) und das verschwenkbare Griffteil (3b) über einen Kopplungsmechanismus lösbar miteinander verbindbar sind, der die Zug-/Schubstange (5) sowohl proximalseitig als auch distalseitig mit Spiel in einer Richtung im wesentlichen quer zur Bewegungsrichtung der Zug-/Schubstange (5) mit den verschwenkbaren Bauteilen (2b, 3b) koppelt,
**dadurch gekennzeichnet,**
**dass** der Kopplungsmechanismus zum lösbaren Verbinden der Zug-/Schubstange (5) mit dem verschwenkbaren Griffteil (3b) als in Langlöchern (7) geführte Schraubverbindung (13) ausgebildet ist.

2. Medizinisches Schneid- und/oder Halteinstrument nach Anspruch 1, **dadurch gekennzeichnet, dass** das stangenseitige Ende des verschwenkbaren Griffteils (3b) zur Aufnahme der Zug-/Schubstange (5) gabelförmig ausgebildet ist.

3. Medizinisches Schneid- und/oder Halteinstrument nach Anspruch 1 oder 2, **dadurch gekennzeichnet, dass** in der Zug-/Schubstange (5) oder im stangenseitigen Ende des verschwenkbaren Griffteils (3b) eine Gewindebuchse zur Aufnahme eines Gewindestifts (14) und im jeweils anderen Bauteil (3b, 5) das Langloch (7) zur führenden Aufnahme des Gewindestifts (14) ausgebildet ist.

4. Medizinisches Schneid- und/oder Halteinstrument nach Anspruch 1, **dadurch gekennzeichnet, dass** zur Ausbildung des beidseitigen Kopplungsspiels die Lagerstelle an jeweils einem der miteinander zu koppelnden Bauteile (5, 2b und 5, 3b) als Langloch (7) ausgebildet ist.

5. Medizinisches Schneid- und/oder Halteinstrument nach Anspruch 4, **dadurch gekennzeichnet, dass** das proximalseitige Langloch (7) als offene Rastnut (9) ausgebildet ist.

6. Medizinisches Schneid- und/oder Halteinstrument nach einem der Ansprüche 1 bis 5, **dadurch gekennzeichnet, dass** Zug-/Schubstange (5) in der mit dem verschwenkbaren Griffteil (3b) gekoppelten Arbeitsstellung über ein Rastelement (11) fixierbar ist.

7. Medizinisches Schneid- und/oder Halteinstrument nach Anspruch 6, **dadurch gekennzeichnet, dass** das Rastelement (11) über ein Federelement (12) in Richtung der verrastenden Stellung vorgespannt ist.

## Claims

1. Medical cutting and/or holding instrument, comprising a shaft (1), a handle (3) that is disposed on the proximal end of the shaft (1) and is composed of two gripping components (3a, 3b) as well as a push/pull rod (5) mounted in a guide groove (4) of the shaft (1), for actuating a tool (2) that is disposed on the distal end of the shaft (1) and composed two jaw components (2a, 2b), wherein for the purpose of opening and closing the tool (2) the push/pull rod (5) is able to be coupled at the distal end to at least one pivotable jaw component (2) of the tool (2) and at the proximal end to a pivotable gripping component (3b) of the handle (3), and wherein the push/pull rod (5) and the pivotable gripping component (3b) are able to be releasably connected to each other through a coupling mechanism that couples the push/pull rod (5) to the pivotable components (2b, 3b) at both the proximal end and the distal end with play in a direction essentially perpendicular to the direction of motion by the push/pull rod (5), **characterized in that** the coupling mechanism is designed as a screw-type attachment (13) guided within slotted holes (7) so as to releasably connect the push/pull rod (5) to the pivotable gripping component (3b).

2. Medical cutting and/or holding instrument according to Claim 1, **characterized in that** the rod-side end of the pivotable gripping component (3b) is fork-shaped so as to receive the push/pull rod (5).

3. Medical cutting and/or holding instrument according to Claims 1 or 2, **characterized in that** in the push/pull rod (5) or in the rod-side end of the pivotable gripping component (3b) a threaded bushing is provided to receive a threaded pin (14) and the slotted hole (7) is provided in the respective other component (3b, 5) to guidingly receive the threaded pin (14).

4. Medical cutting and/or holding instrument according to Claim 1, **characterized in that** the mounting point is provided in the form of a slotted hole (7) on each of the components (5, 3b) to be coupled together so as provide mutual coupling play.

5. Medical cutting and/or holding instrument according to Claim 4, **characterized in that** the proximal-side slotted hole (7) is in the form of a detent groove (9).

6. Medical cutting and/or holding instrument according to one of Claims 1 through 5, **characterized in that** push/pull rod (5) is able to be secured by a detent element (11) when in the operating position coupled to the pivotable gripping component (3b).

7. Medical cutting and/or holding instrument according to Claim 6, **characterized in that** the detent element (11) is preloaded by a spring element (12) in the direction of the locking position.

## Revendications

1. Instrument médical de coupe et/ou de maintien comprenant un corps allongé (1), une poignée de manoeuvre (3) agencée à l'extrémité proximale du corps allongé (1) et constituée de deux pièces de préhension (3a, 3b), ainsi qu'une tige de traction/poussée (5) montée dans une rainure de guidage (4) du corps allongé (1) et destinée à actionner un outil (2) agencé à l'extrémité distale du corps allongé (1) et constitué de deux pièces de mâchoire (2a, 2b), la tige de traction/poussée (5) pouvant, pour l'ouverture et la fermeture de l'outil (2), être couplée, côté distal, à au moins une pièce de mâchoire (2b) pivotante de l'outil (2), et, côté proximal, à une pièce de préhension pivotante (3b) de la poignée de manoeuvre (3), et la tige de traction/poussée (5) et la pièce de préhension pivotante (3b) pouvant être reliées l'une à l'autre de manière amovible par l'intermédiaire d'un mécanisme de couplage, qui assure le couplage de la tige de traction/poussée (5), aussi bien côté proximal que côté distal, aux pièces pivotantes (2b, 3b), avec jeu dans une direction sensiblement transversale à la direction de mouvement de la tige de traction/poussée (5),
**caractérisé en ce que** le mécanisme de couplage pour relier de manière amovible la tige de traction/poussée (5) à la pièce de préhension pivotante (3b), est réalisé en tant que liaison à vis (13) guidée dans des trous oblongs (7).

2. Instrument médical de coupe et/ou de maintien selon la revendication 1, **caractérisé en ce que** l'extrémité côté tige de la pièce de préhension pivotante (3b) est d'une configuration en forme de fourchette, en vue de recevoir la tige de traction/poussée (5).

3. Instrument médical de coupe et/ou de maintien selon la revendication 1 ou la revendication 2, **caractérisé en ce que** dans la tige de traction/poussée (5), ou bien dans l'extrémité côté tige de la pièce de préhension pivotante (3b), est formée une douille filetée destinée à recevoir une broche filetée (14), et respectivement dans l'autre pièce (3b, 5) est formé le trou oblong (7) pour y recevoir, en la guidant, la broche filetée (14).

4. Instrument médical de coupe et/ou de maintien selon la revendication 1, **caractérisé en ce que** pour former le jeu de couplage des deux côtés, la zone de palier est réalisée respectivement sur l'une des pièces à coupler (5, 2b et 5, 3b), en tant que trou oblong (7).

5. Instrument médical de coupe et/ou de maintien selon la revendication 4, **caractérisé en ce que** le trou oblong (7), côté proximal, est réalisé en tant que rainure d'encliquetage (9) ouverte.

6. Instrument médical de coupe et/ou de maintien selon l'une des revendications 1 à 5, **caractérisé en ce que** la tige de traction/poussée (5) peut être fixée dans la position de travail couplée à la pièce de préhension pivotante (3b), par l'intermédiaire d'un élément d'encliquetage (11).

7. Instrument médical de coupe et/ou de maintien selon la revendication 6, **caractérisé en ce que** l'élément d'encliquetage (11) est précontraint en direction de la position où se produit l'encliquetage, par l'intermédiaire d'un élément de ressort (12).
